(19) Europäisches Patentamt
European Patent Office
Office européen des brevets

(11) Publication number : **0 357 192 B1**

# (12) EUROPEAN PATENT SPECIFICATION

(45) Date of publication of patent specification : **06.05.92 Bulletin 92/19**

(51) Int. Cl.$^5$ : **C07C 335/06**

(21) Application number : **89306802.3**

(22) Date of filing : **04.07.89**

## (54) Process for preparing substituted guanylthioureas.

(30) Priority : **21.07.88 US 223132**

(43) Date of publication of application : **07.03.90 Bulletin 90/10**

(45) Publication of the grant of the patent : **06.05.92 Bulletin 92/19**

(84) Designated Contracting States : **AT BE CH DE ES FR GB GR IT LI LU NL SE**

(56) References cited : **US-A- 4 560 690**

(73) Proprietor : **PFIZER INC.**
**235 East 42nd Street**
**New York, N.Y. 10017 (US)**

(72) Inventor : **Reiter, Lawrence Alan**
**32, West Mystic Avenue**
**Mystic Connecticut (US)**

(74) Representative : **Wood, David John et al**
**PFIZER LIMITED, Ramsgate Road**
**Sandwich, Kent CT13 9NJ (GB)**

EP 0 357 192 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is directed to the advantageous process for the preparation of substituted guanylthioureas, which are intermediates in the synthesis of 2-(1-alkyl-3-guanidino)-4-(2-methyl-4-imidazolyl)thiazole antiulcer agents.

U.S. Patent 4,560,690 describes the synthesis of 2-(1-n-pentyl-3-guanidino)-4-(2-methyl-4-imidazolyl) thiazole and various analogs, having valuable antiulcer activity. This patent, which includes pharmaceutical compositions and a method of inhibiting ulcers with these compounds, reports the preparation of these agents by the following scheme:

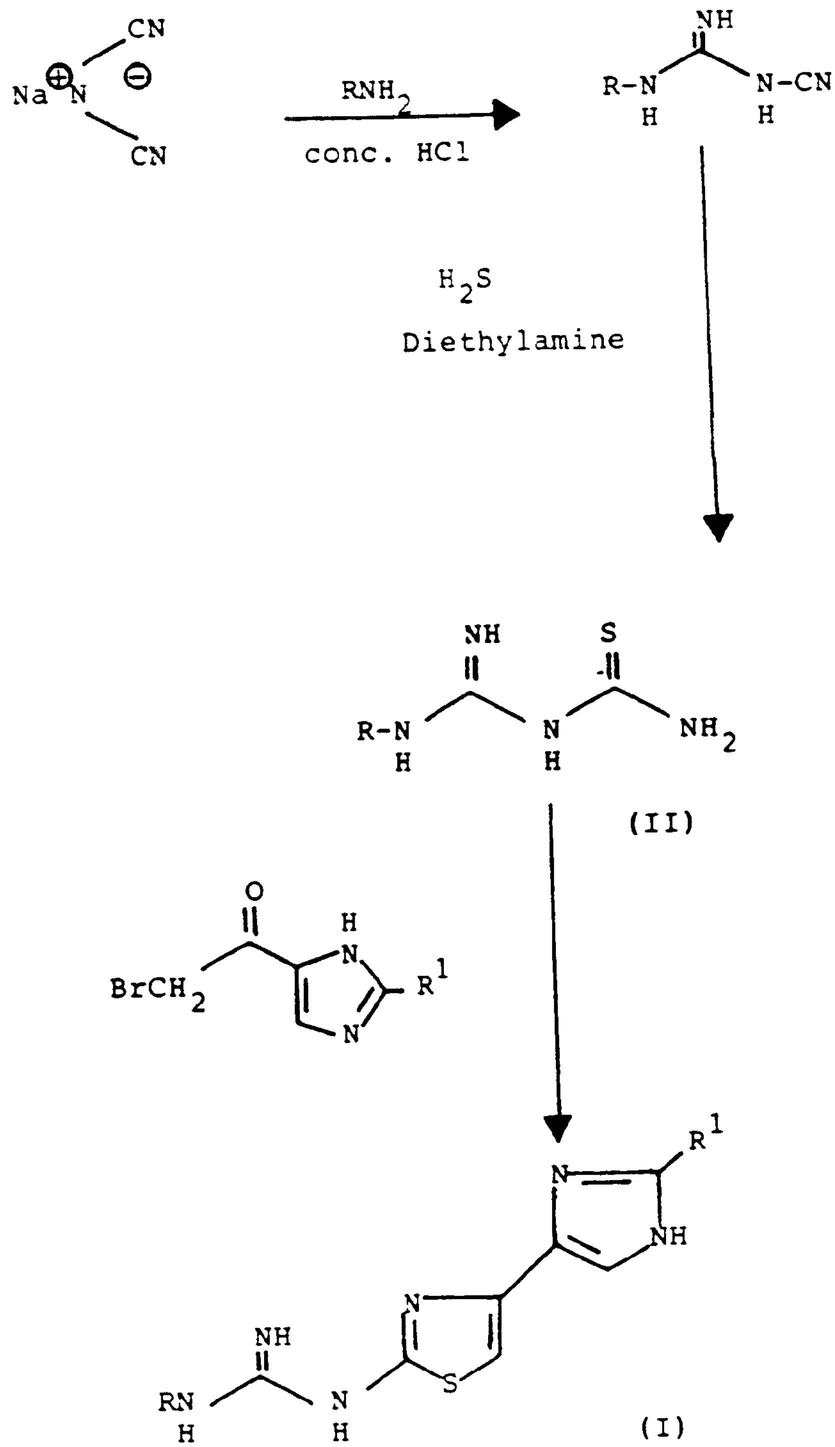


(II)

(I)

wherein R is benzyl or ($C_1$-$C_6$)alkyl and $R^1$ is ($C_1$-$C_5$)alkyl.

The synthesis of the intermediates in this reaction, namely II, employs reagents which are very toxic, especially hydrogen sulfide. In particular, the use of large excesses of hydrogen sulfide (or alternately hydrogen sulfide under pressure) presents particular problems which limit batch sizes.

An alternate method for preparing compounds of formula II is reported in International Application No. PCT/US86/02308 which comprises an amine exchange with amidinothiourea by the following scheme:

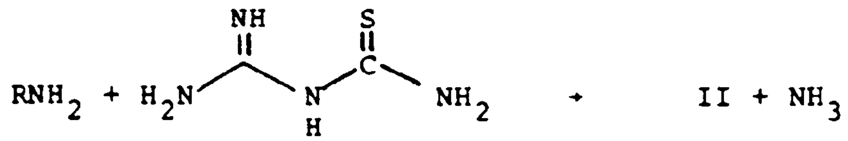

This route avoids the use of toxic hydrogen sulfide.

## Summary of the Invention

It has now been found that a further route to II exists which not only avoids the use of hydrogen sulfide but provides II in isolated yields considerably higher than those reported in our herein cited International Application. This process consists of heating a compound of the formula

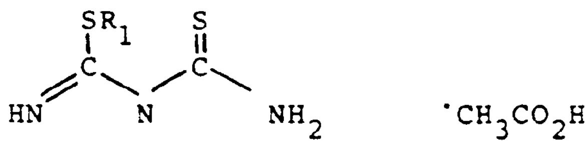

where $R_1$ is alkyl of one to three carbon atoms with at least one molar equivalent of an amine of the formula

$$R\text{-}NH_2$$

where R is alkyl of one to six carbon atoms or benzyl in a reaction-inert solvent.

A preferred embodiment of this process is the reaction of those compounds where R is n-pentyl and $R_1$ is methyl. A further embodiment is the use of an alkanol as the reaction-inert solvent. Especially preferred is i-propanol as said solvent.

The expression "reaction-inert solvent" refers to a solvent which does not interact with starting material, intermediates or products in a manner which adversely affects the yield or purity of the desired product.

## Detailed Description of the Invention

The process of the present invention is readily carried out by treating an S-alkyldithiobiuret acetate, formed by reacting an acid addition salt, such as a hydrogen iodide, with at least an equimolar amount of sodium acetate, with at least an equimolar amount of an amine $RNH_2$. As much as a 20 percent excess of sodium acetate and 10-20 per cent excess of amine can be employed without affecting the course of reaction or purity of the product.

The solvent for this reaction should be a reaction-inert solvent as previously defined, as well as one which allows the reaction to be heated in the range of 65-100°C., and one which at least partially solibilizes the reactants. Such solvents include alkanols of one to three carbon atoms.

The reaction times will vary with the inherent reactivity of the starting reagents, but at the reflux temperature of the preferred solvents, the reaction should be complete in about 24 hours.

The product, as the free base, is isolated by removal of the reaction solvent followed by partitioning the residual material between aqueous sodium bicarbonate and a water immiscible solvent such as chloroform. The organic phase is dried and the solvent removed to give the final product.

The product, if needed, can be purified by flash chromatography.

The following examples are given by way of illustration and are not to be construed as limitation of this invention.

EXAMPLE 1

S-Methyldithiobiuretium iodide

Prepared by modification of the procedure of K. Jensen and P. H. Nielsen (Acta Chem. Scand. 1966, 20, 597): Dithiobiuret (10.14 g, 75.00 mmole) was dissolved in THF (150 mL) and treated with excess methyl iodide (26.54 g, 187.5 mmole) in one portion. After stirring for 24 hrs. at room temperature, the mixture was diluted with toluene (300 mL) to precipitate the product. The resulting slurry was chilled for a few hours then the solid was collected and washed well with cold toluene: THF (2:1). Drying gave 18.33 g (88%) of light yellow sold: mp 135°C. softens, 142-144°C. melts (lit. 132-134°C.); mass spectrum m/e (relative intensity) $M^+$, 149 (13.4), 134 (100).

EXAMPLE 2

(N-n-Pentylguanyl)thiourea (R=n-$C_5H_{11}$)

S-Methyldithiobiuretium iodide (5.54 g, 20.0 mmole), sodium acetate (1.80 g., 22.0 mmole) and pentylamine (1.83 g, 21.0 mmole) were refluxed together in 2-propanol (100 mL). After 22 hrs., the warm mixture was filtered to remove some suspended material and the solvent removed in vacuo. The residue was basified with saturated sodium bicarbonate solution (100 mL) and extracted with chloroform (3 x 50 mL). The combined extracts were dried with magnesium sulfate, filtered and concentrated to give 3.36 g (89%) of a light yellow solid. This was flash chromatographed (5:95 - methanol: chloroform) to give 2.55 g (68%) of colorless solid. A 200 mg portion was recrystallized from cyclohexane (10 mL)/ethyl acetate to give 136 mg of fine colorless needles: mp 102-104°C.; mass spectrum m/e (relative intensity) $M^+$, 188(100), 155 (47.2); $^1$H-NMR ($CDCl_3$) δ 0.82 (3H, t), 1.3-1.6 (6H, m), 3.15 (2H, t). Anal. Calc'd for $C_7H_{16}N_4S$: C, 44.65; H, 8.57; N, 29.76. Found: C, 44.72; H, 8.21; N, 29.62.

EXAMPLE 3

(N-n-Hexylguanyl)thiourea (R=n-$C_6H_{13}$)

S-Methyldithiobiuretium iodide (5.54 g, 20.0 mmole), sodium acetate (1.80 g, 22.0 mmole) and n-hexylamine (2.12 g, 21.0 mmole) were refluxed together in 2-propanol (100mL). After 23 hrs., the warm mixture was filtered to remove some suspended material and the solvent removed in vacuo. The residue was basified with saturated sodium bicarbonate solution (100 mL) and extracted with chloroform (3 x 50 mL). The combined extracts were dried with magnesium sulfate, filtered and concentrated to give 4.10 g (100%) of a light yellow solid. This was flash chromatographed (5:95 - methanol: chloroform) to give 3.03 g (75%) of colorless solid. A 200 mg portion was recrystallized from cyclohexane (10 mL)/ethyl acetate to give 150 mg of fluffy white solid: mp 75-76°C.; mass spectrum m/e (relative intensity) $M^+$, 202 (100), 169 (43.5); $^1$H-NMR ($CDCl_3$) δ 0.85 (3H, br t), 1.26 (6H, br s), 1.43 (2H, br s), 3.09 (2H, q). Anal. Calc'd for $C_8H_{18}N_4S$ : C, 47.49; H, 8.97; N, 27.69. Found: C, 47.81; H, 9.12; N, 27.46.

EXAMPLE 4

(N-Benzylguanyl)thiourea (R=$C_6H_5CH_2$)

S-Methyldithiobiuretium iodide (5.54 g, 20.0 mmole), sodium acetate (1.80 g, 22.0 mmole) and benzylamine (2.25 g, 21.0 mmole) were refluxed together in 2-propanol (100 mL). After 23 hrs., the warm mixture was filtered to remove some suspended material and the mixture cooled to 5°C. overnight. The resulting precipitate was collected, washed with cold 2-propanol and dried yielding 3.19 g (59%) of the acetate salt of the product as a colorless powder. The filtrate from the acetate salt was concentrated in vacuo. The residue was basified with saturated sodium bicarbonate solution (100 mL) and extracted with chloroform (3 x 50 mL). The combined extracts were dried with magnesium sulfate, filtered and concentrated to give 1.62 g (39%) of a light orange oil. This was flash chromatographed (5:95 - methanol: chloroform) to give 679 mg (16%) of pale yellow solid. Total yield: 75%. A 200 mg portion of the acetate salt was recrystallized from 2-propanol (5 mL) to give 110 mg of fluffy white solid: mp 157-159°C.; mass spectrum m/e (relative intensity) $M^+$, 208 (73.1), 175 (85.4), 91 (100); $^1$H-NMR ($d_6$-$Me_2SO$) δ 1.87 (3H, s), 4.38 (2H, s), 7.27 (5H, br s). Anal. Calc'd for $C_9H_{12}N_4S \cdot C_2H_4O$: C, 49.23; H, 6.01; N, 20.88. Found: C, 49.09; H, 6.15; N, 20.64.

EXAMPLE 5

(N-Phenylguanyl)thiourea ($R=C_6H_5$)

S-Methyldithiobiuretium iodide (1.11 g, 4.0 mmole), sodium acetate (361 mg, 4.4 mmole) and aniline (391 mg, 4.2 mmole) were refluxed together in 2-propanol (40 mL). After 23 hrs., a second portion of aniline (75 mg, 0.2 equivalents) was added and reflux continued. After another 24 hrs., the solvent was removed in vacuo. The residue was basified with saturated sodium bicarbonate solution (50 mL) and extracted with chloroform (4 x 25 mL). The combined extracts were dried with magnesium sulfate, filtered and concentrated to give 780 mg (100%) of a pale yellow oil. This was flash chromatographed (5:95-methanol: chloroform) to give 221 mg (28%) of pure product which was then recrystallized from ethyl acetate (10 mL) to give 122 mg of chunky colorless crystals: mp 153-155°C.; mass spectrum m/e (relative intensity) $M^+$, 194 (100), 161 (38.8); $^1H$-NMR ($CD_3OD$) δ 7.13 (1H, br t), 7.24 (2H, m), 7.34 (2H, t). Anal. Calc'd for $C_8H_{10}N_4S$: C, 49.46; H, 5.19; N, 28.84. Found: C, 49.16; H, 5.21; N, 28.81.

## Claims

1. A process for preparing a compound of the formula

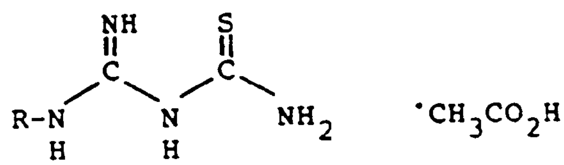

wherein R is alkyl having one to six carbon atoms or benzyl, which comprises heating an S-alkyldithiobiuretium of the formula

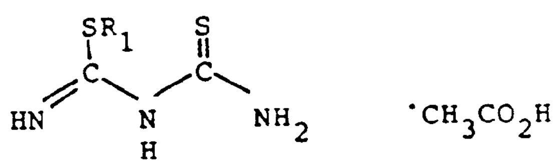

wherein $R_1$ is alkyl having one to three carbon atoms with at least one molar equivalent of an amine of the formula

$$R\text{-}NH_2$$

in a reaction-inert solvent.

2. A process of claim 1, wherein R is n-pentyl and $R_1$ is methyl.

3. A process of claim 1 or 2, wherein the reaction-inert solvent is an alkanol having one to three carbon atoms.

4. The process of claim 3, wherein the reaction-inert solvent is i-propanol.

## Patentansprüche

1. Verfahren zur Herstellung einer Verbindung der Formel

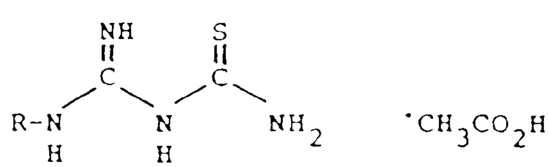

worin R Alkyl mit 1 bis 6 Kohlenstoffatomen oder Benzyl bedeutet, das das Erhitzen eines S-Alkyldithiobiurets der Formel

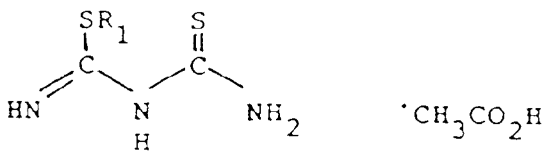

,

worin $R_1$ Alkyl mit 1 bis 3 Kohlenstoffatomen darstellt, mit wenigstens einem Moläquivalent eines Amins der Formel

$$R\text{-}NH_2$$

in einem reaktionsinerten Lösungsmittel umfaßt.

2. Verfahren nach Anspruch 1, in dem R n-Pentyl und $R_1$ Methyl bedeutet.

3. Verfahren nach Anspruch 1 oder 2, in dem das reaktionsinerte Lösungsmittel ein Alkanol mit 1 bis 3 Kohlenstoffatomen ist.

4. Verfahren nach Anspruch 3, in dem das reaktionsinerte Lösungsmittel Isopropanol ist.

## Revendications

1. Procédé de préparation d'un composé de formule

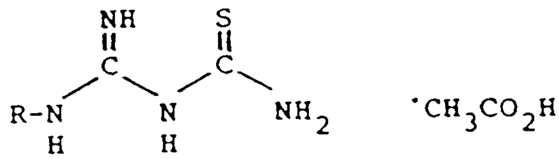

dans laquelle R représente un groupe alkyle ayant 1 à 6 atomes de carbone ou benzyle, qui consiste à chauffer un S-alkyldithiobiurétium de formule

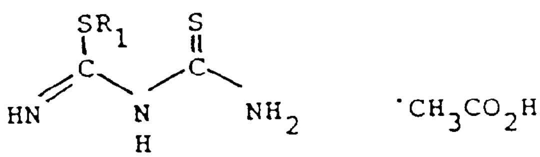

dans laquelle $R_1$ représente un groupe alkyle ayant 1 à 3 atomes de carbone, avec au moins un équivalent molaire d'une amine de formule

$$R\text{-}NH_2$$

dans un solvant inerte vis-à-vis de la réaction.

2. Procédé suivant la revendication 1, dans lequel R représente un groupe n-pentyle et $R_1$ représente un groupe méthyle.

3. Procédé suivant la revendication 1 ou 2, dans lequel le solvant inerte vis-à-vis de la réaction est un alcanol ayant 1 à 3 atomes de carbone.

4. Procédé suivant la revendication 3, dans lequel le solvant inerte vis-à-vis de la réaction est l'isopropanol.